# EUROPEAN PATENT APPLICATION

(11) **EP 2 923 592 A1**
(43) Date of publication of application: **30.09.2015**
(21) Application number: 14162127.6
(22) Date of filing: 27.03.2014
(51) Int. Cl.: A41D 19/015, F24H 1/20, H05B 1/00, A61F 7/00

(54) **Heating device with hand warming structure**

(71) Applicant: Liang, Shengquan, Foshan, Guangdong 528300 (CN)
(72) Inventor: Liang, Shengquan, Foshan, Guangdong 528300 (CN)
(74) Representative: Arpe Fernandez, Manuel de

(57) **Abstract**

The invention provides a heating device with hand warming structure including a heating device body and a hand warming structure. The hand warming structure is disposed outside the heating device body and there are openings for the extension of users' hands at the two ends thereof. The hand warming structure and the heating device body comprise a heating cavity. The invention has advantages of easy holding, comfort and practicality, heat locking and enhanced heating effect.

## Description

### FIELD OF THE INVENTION

The present invention relates to the field of heating device, and particularly to a heating device with hand warming structure.

### BACKGROUND OF THE INVENTION

In the cold winter, many people may use some heating devices to get warm. There is a hot water warmer which is easy to carry. The hot water warmer is made of water-proof material to a sealed warmer body, the internal cavity of which is used for storing hot water. The heat is transmitted by the hot water to the warmer body, and then the heat is transmitted to human body through the warmer body, thus heating effect being achieved. The structure of such hot water warmer is simple. When the warmer body of such hot water warmer is grabbed, the palm of the hand may contact with the warmer body, but the back of the hand is exposed to the cold air, so that the heating effect is significantly reduced.

Meanwhile, when in use, users need to hold the warmer body with hand and grabs it to the part wanted to be heated, such as abdomen. Once the hand releases, the hot water warmer will fall down from users' body. The warmer body is made of thick, high temperature resistant material, the texture of which is hard, making human body uncomfortable when contacts with it. And such material causes the heat of the hot water warmer to rapidly dissipate, so the heating effect can not sustain for a long time.

It is not difficult to see that there are some drawbacks in the prior art.

### SUMMARY OF THE INVENTION

The invention provides a heating device with hand warming structure which has advantages of easy holding, comfort and practicality, heat locking and enhanced heating effect.

In order to achieve the above aims, the invention employs the following technical solution:
A heating device with hand warming structure includes a heating device body and a hand warming structure. The hand warming structure is disposed outside the heating device body and there are openings for the extension of users' hands at the two ends thereof. The hand warming structure and the heating device body comprise a heating cavity.

Further, the heating device body is an electric hot water warmer, and the hand warming structure is disposed outside the electric hot water warmer.

Further, the heating device body is an accommodating device with an opening at one end and an electric hot water warmer. The electric hot water warmer is placed inside the accommodating device, and the hand warming structure is provided outside the accommodating device.

Further, the accommodating device is a cloth case with zip.

Further, the fabric of the heating device body is flexible fabric.

Further, the fabric of the heating device body is plush fabric.

Further, the fabric of the hand warming structure is flexible fabric.

Further, the fabric of the hand warming structure is plush fabric.

Further, the electric hot water warmer includes a sealed warmer body, a heating unit and a power unit. A liquid medium for transmitting heat is filled in a sealed cavity inside the sealed warmer body. The total volume of the liquid medium when expanding or vaporized under predetermined temperature corresponds to the normal volume of the liquid medium under room temperature. The heating unit is disposed in the sealed cavity and immersed in the liquid medium. The power unit is sealingly connected with the sealed warmer body. A part of the power unit is exposed in the exterior of the sealed warmer body, and another part thereof is sealed in the sealed cavity of the sealed warmer body. The power unit is electrically connected with the heating unit.

Further, the sealed warmer body includes an inner warmer body and an outer warmer body. The inner warmer body is located at the innermost layer of the sealed warmer body and it is made of flexible waterproof material. The sealed cavity formed by the inner warmer body is used to store the liquid medium. The outer warmer body is located at the outermost layer of the sealed warmer body and it is made of flexible material. The outer warmer body is intimately fitted with the outer warmer body. The outer warmer body is used for the contact with human body.

Further, the power unit includes a mounting sleeve, a temperature controlling unit and a socket. The mounting sleeve includes an external portion and an internal portion. The mounting sleeve is sealingly connected with the sealed warmer body. The external portion of the mounting sleeve is exposed to the exterior of the sealed warmer body, and the internal portion of the mounting sleeve is sealed in the sealed cavity of the sealed warmer body. A temperature controlling unit is provided in the internal portion of the mounting sleeve and is cascaded with the heating unit. The temperature controlling unit is used for controlling the conduction and the breakage of the circuit according to the internal temperature of the heating warmer. The socket is mounted on the external portion of the mounting sleeve and is exposed to the exterior of the sealed warmer body. The socket is electrically connected with the temperature controlling unit and the heating unit, constituting a complete circuit.

Further, a heating pipe is provided inside the hand warming structure. The heating pipe is communicated with the sealed cavity.

The heating device with hand warming structure provided by the invention has the following advantages:
When the heating device is used, users can extend hands into the heating cavity from openings. The heat of the heating device body can be effectively locked in the heating cavity and thus the whole hand can be heated, the heating effect being enhanced;

The hand warming structure surrounds users' hands, so users do not have to additionally hold the heating device body;

The heating device body and the hand warming structure are made of flexible fabric, so the texture is soft and they are beneficial to the contact with human body, enhancing the comfort of users;

The heating device body and the hand warming structure are made of plush fabric. Fine fluff on the plush fabric can further help to store the heat and thus the dissipation of the heat is reduced so that the heating effect is sustained longer.

### BRIEF DESCRIPTION OF THE DRAWINGS

In order to more clearly illustrate embodiments of the invention or the technical solutions in the prior art, there is provided a brief introduction to the accompanying drawings which are necessary in the description of the embodiments or the prior art below. Obviously, the drawings described below are only a few embodiments of the invention. For those ordinary skilled in the art, other drawings may be obtained according to these drawings under the premise that creative work is not done.
Figure 1 is a structural schematic view of a heating device with hand warming structure provided by the embodiment of the invention;
Figure 2 is a side view of Figure 1;
Figure 3 is a structural schematic view of the heating device body;
Figure 4 is a structural schematic view of the heating device body with an indicator light;

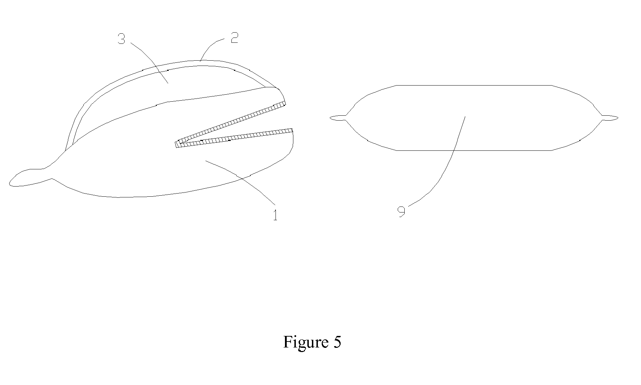

Figure 5 is a structural schematic view of the second embodiment of the invention.

Illustration of the reference numeral

| | |
|---|---|
| 1-accommodating device | 2-hand warming structure |
| 3-heating cavity | 4-power unit |
| 5-heating unit | 6-outer warmer body |
| 7-inner warmer body | 8-indicator light |
| 9-electric hot water warmer | |

### DETAILED DESCRIPTION

In order to make the purpose, technical solutions and advantages of the embodiments more clear, the technical solutions in the embodiments of the invention will be described clearly and completely below in combination with the embodiments and drawings of the invention. It should be explained that the described embodiments are only a part of embodiments of the invention not all embodiments. Based on the embodiments of the invention, all other embodiments obtained by those ordinary skilled under the premise that no creative work is done fall in the protection scope of the invention.

### First embodiment

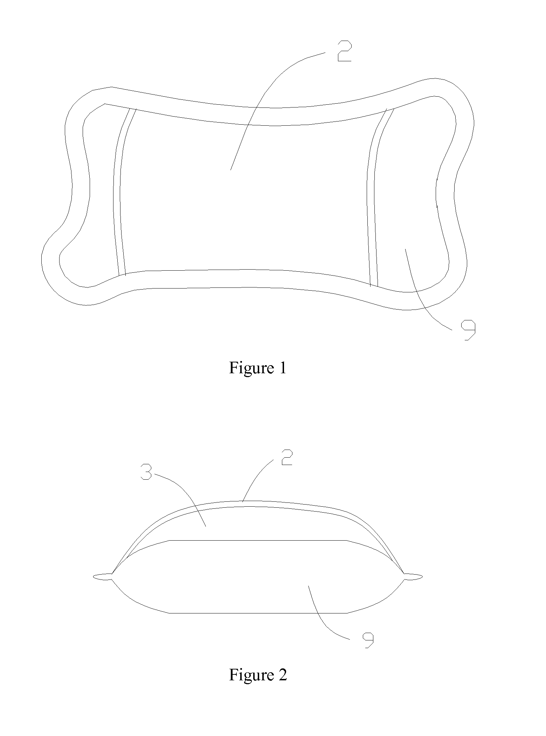

Reference is made to Figures 1 and 2. A heating device with hand warming structure includes a heating device body and a hand warming structure 2. The hand warming structure 2 is disposed outside the heating device body and there are openings for the extension of users' hands at the two ends thereof. The hand warming structure 2 and the heating device body comprise a heating cavity 3.

In the present embodiment, the heating device body is an electric hot water warmer. The hand warming structure 2 is disposed outside the electric hot water warmer 9.

When the heating device of the invention is used, users may extend their hands into the heating cavity 3 from openings. The heating warmer in the prior art has no hand warming structure 2. When users' hands put on the surface of the heating warmer, although palms can be heated, the back of hands exposed to cold air is completely not heated. Different from the prior art, the hand warming structure 2 of the invention may effectively lock the heat in the heating cavity 3 so that the hands placed in the heating cavity 3 can be heated well.

Meanwhile, the hand warming structure 2 can surround users' hands and thus holds users' hands so that the heating device body can follow the movement of users' hands and is close to users' hands all the time. Therefore, users do not have to additionally hold the heating device body, which facilitates the use of the heating warmer.

As an improvement, the fabrics of the heating device body and the hand warming structure 2 are flexible fabrics. The fabric of the hot water warmer in the prior art is a thick, high temperature resistant material, the texture of which is hard, making human body uncomfortable when contacts with it. In the invention, the fabric is improved to a flexible fabric, making users comfortable.

As an improvement, the fabrics of the heating device body and the hand warming structure 2 are plush fabrics. If the flexible material whose surface texture is smooth is only used, the heat of the heating warmer is rapidly dissipated so that the continuous heating cannot be obtained. Fine fluff on the surface of the plush fabric can effectively store the heat and thus the dissipation of the heat is reduced so that the heating effect of the heating warmer is sustained longer.

The heating device with hand warming structure provided by the invention has a newly added hand warming structure 2 so that it users can extend their hands into the heating cavity 3 comprised of the hand warming structure 2 and the heating device body from openings. In the condition that the heat of the heating cavity 3 is locked, users can be heated well. Meanwhile, the hand warming structure 2 can surround users' hands and thus holds users' hands so that users do not have to additionally hold the heating warmer, which facilitates the use of the heating device. In the invention, the heating warmer is further improved in the choice of the fabric, which is beneficial to the contact with human body, enhances the comfort of users, and in the meantime helps to store the heat and reduce the dissipation of the heat so that the heating effect is sustained longer.

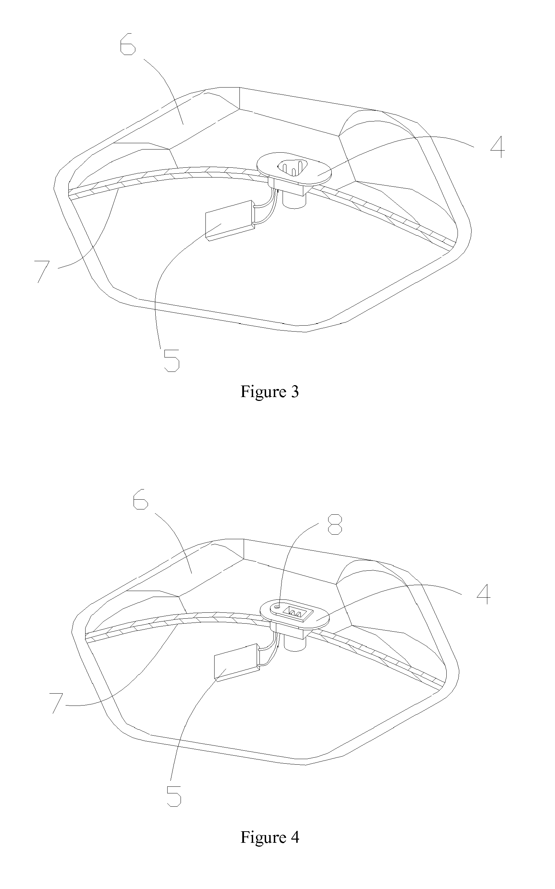

Reference is made to Figures 3 and 4. The heating device includes a sealed warmer body, a heating unit 5 and a power unit 4. A liquid medium for transmitting heat is filled in a sealed cavity inside the sealed warmer body. The total volume of the liquid medium when expanding or vaporized under predetermined temperature corresponds to the normal volume of the liquid medium under room temperature. The heating unit 5 is disposed in the sealed cavity and immersed in the liquid medium. The power unit 4 is sealingly connected with the sealed warmer body. A part of the power unit 4 is exposed in the exterior of the sealed warmer body, and another part thereof is sealed in the sealed cavity of the sealed warmer body. The power unit 4 is electrically connected with the heating unit 5.

Particularly, the sealed warmer body includes an inner warmer body 7 and an outer warmer body 6. The inner warmer body 7 is located at the innermost layer of the sealed warmer body and it is made of flexible waterproof material. The sealed cavity formed by the inner warmer body 7 is used to store the liquid medium. The outer warmer body 6 is located at the outermost layer of the sealed warmer body and it is made of flexible material. The outer warmer body 6 is intimately fitted with the outer warmer body 7. The outer warmer body 6 is used for the contact with human body.

Two-layer design being used in the sealed warmer body is due to different requirements for the outer and inner material textures of the sealed warmer body. The inner warmer body 7 seals the liquid medium inside the sealed warmer body to ensure that the liquid medium may not leak in the process of the use. The material texture of the outer warmer body 6 is the fabric of the above mentioned heating device, and this mainly takes the comfort of users into account so the detailed function thereof is no described.

Particularly, the power unit 4 includes a mounting sleeve, a temperature controlling unit and a socket. The mounting sleeve includes an external portion and an internal portion. The mounting sleeve is sealingly connected with the sealed warmer body. The external portion of the mounting sleeve is exposed to the exterior of the sealed warmer body, and the internal portion of the mounting sleeve is sealed in the sealed cavity of the sealed warmer body. A temperature controlling unit is provided in the internal portion of the mounting sleeve and is cascaded with the heating unit 5.The temperature controlling unit is used for controlling the conduction and the breakage of the circuit according to the internal temperature of the heating warmer. The socket is mounted on the external portion of the mounting sleeve and is exposed to the exterior of the sealed warmer body. The socket is electrically connected with the temperature controlling unit and the heating unit 5, constituting a complete circuit.

When the electricity is fed into the power unit 5 through a socket, the power unit 5 may generate heat to heat the liquid medium. Thus users do not have to heat the liquid medium or to replace it so that the operation is convenient and the practicality is strong. Meanwhile, the temperature controlling unit can control the conduction and the breakage of the circuit through the temperature of the liquid medium. Once the liquid medium is heated to a certain temperature, the temperature controlling unit will automatically break the circuit and thus the heating is stopped, preventing that the liquid medium is overheated. Thereby, the safety is very high.

As an improvement, the power unit 4 further includes an indicator light 8. The indicator light 8 is mounted on the external portion of the mounting sleeve and is exposed to the exterior of the sealed warmer body. The indicator light 8 is paralleled with the heating unit 5. The indicator light 8 can make users clearly know the working state of the heating warmer.

As an improvement, the power unit 4 further includes a cover. The cover is disposed on the socket and is movably connected with the power unit 4 for controlling the exposure and the closing of the socket. The cover is mainly used for protecting the socket, preventing the damage of the socket and the entrance of foreign matter. Thereby, the using life of the heating warmer may be extended.

As an improvement, a heating pipe is provided inside the hand warming structure 2. The heating pipe is communicated with the sealed cavity and is preferably a plastic tube of small diameter. The heating pipe can not only further enhance the heating effect, but also the structure is simple and thus it is easy to manufacture with a lower production cost.

### Second embodiment

The present embodiment is different from the first embodiment only in that the heating device body is an accommodating device 1 with an opening at one end and an electric hot water warmer 9. The electric hot water warmer 9 is placed inside the accommodating device 1. The hand warming structure 2 is provided outside the accommodating device 1.

The split of the electric hot water warmer 9 and the accommodating device 1 is in favor of the clearing of the accommodating device 1. In principle, the electric hot water warmer 9 is difficult to clear. After a long period of using, the heating device body is inevitably soiled. As described in the first embodiment, the electric hot water warmer 9 is directly used as the heating device body, which will cause the problem that the stain is difficult to remove. As described in the present embodiment, the heating device body is divided into two parts, namely the accommodating device 1 and the electric hot water warmer 9. When the heating device is used, the electric hot water warmer 9 is placed in the accommodating device 1. After a long period of using, the surface of the accommodating device 1 is soiled. At this moment, the electric hot water warmer 9 may be directly taken out and the accommodating device 1 is solely cleaned.

The accommodating device 1 is a cloth case with zip. The fabric of the accommodating device 1 is the fabric of the heating device body. The technical characteristic and the function thereof is the same as the first embodiment and it will not be repeated.

The above-mentioned embodiments are only several embodiments of the invention. The description of the above-mentioned embodiments is more specific and detail, but they should not be considered to be a limitation of the invention. It should be noted that several variations and improvements may also be made without departing the concept of the invention for those ordinary skilled in the art. These variations and improvements all belong to the protection scope of the invention. Therefore, the protection of the invention should take the appended claim as standard.

## Claims

1. A heating device with hand warming structure comprising a heating device body and a hand warming structure, wherein the hand warming structure is disposed outside the heating device body and there are openings for the extension of users' hands at the two ends thereof, the hand warming structure and the heating device body comprise a heating cavity.

2. The heating device with hand warming structure according to claim 1, wherein the heating device body is an electric hot water warmer and the hand warming structure is disposed outside the electric hot water warmer.

3. The heating device with hand warming structure according to claim 1, wherein the heating device body is an accommodating device with an opening at one end and an electric hot water warmer, the electric hot water warmer is placed inside the accommodating device and the hand warming structure is provided outside the accommodating device.

4. The heating device with hand warming structure according to claim 3, wherein the accommodating device is a cloth case with zip.

5. The heating device with hand warming structure according to claim 1, wherein the fabric of the heating device body is flexible fabric.

6. The heating device with hand warming structure according to claim 5, wherein the fabric of the heating device body is plush fabric.

7. The heating device with hand warming structure according to claim 1, wherein the fabric of the hand warming structure is flexible fabric.

8. The heating device with hand warming structure according to claim 7, wherein the fabric of the hand warming structure is plush fabric.

9. The heating device with hand warming structure according to claim 2, wherein the electric hot water warmer comprises a sealed warmer body, a heating unit and a power unit, a liquid medium for transmitting heat is filled in a sealed cavity inside the sealed warmer body, the total volume of the liquid medium when expanding or vaporized under predetermined temperature corresponds to the normal volume of the liquid medium under room temperature, the heating unit is disposed in the sealed cavity and immersed in the liquid medium, the power unit is sealingly connected with the sealed warmer body, a part of the power unit is exposed in the exterior of the sealed warmer body, another part thereof is sealed in the sealed cavity of the sealed warmer body and the power unit is electrically connected with the heating unit.

10. The heating device with hand warming structure according to claim 9, wherein the sealed warmer body comprises an inner warmer body and an outer warmer body, the inner warmer body is located at the innermost layer of the sealed warmer body and it is made of flexible waterproof material, the sealed cavity formed by the inner warmer body is used to store the liquid medium, the outer warmer body is located at the outermost layer of the sealed warmer body and it is made of flexible material, the outer warmer body is intimately fitted with the outer warmer body and the outer warmer body is used for the contact with human body.

11. The heating device with hand warming structure according to claim 9, wherein the power unit comprises a mounting sleeve, a temperature controlling unit and a socket, the mounting sleeve comprises an external portion and an internal portion, the mounting sleeve is sealingly connected with the sealed warmer body, the external portion of the mounting sleeve is exposed to the exterior of the sealed warmer body, and the internal portion of the mounting sleeve is sealed in the sealed cavity of the sealed warmer body, a temperature controlling unit is provided in the internal portion of the mounting sleeve and is cascaded with the heating unit, the temperature controlling unit is used for controlling the conduction and the breakage of the circuit according to the internal temperature of the heating warmer, the socket is mounted on the external portion of the mounting sleeve and is exposed to the exterior of the sealed warmer body and the socket is electrically connected with the temperature controlling unit and the heating unit, constituting a complete circuit.

12. The heating device with hand warming structure according to claim 9, wherein a heating pipe is provided inside the hand warming structure and the heating pipe is communicated with the sealed cavity.
